# DEMANDE DE BREVET EUROPEEN

(11) **EP 3 492 015 A1**
(43) Date de publication de la demande: **05.06.2019**
(21) Numéro de dépôt: 18208815.3
(22) Date de dépôt: 28.11.2018
(51) Int. Cl.: A61B 5/18, G06K 9/00

(54) **DISPOSITIF ET PROCEDE DE DETECTION D'EMOTION**

(30) Priorité: 29.11.2017 FR 1761351
(71) Demandeur: Valeo Comfort and Driving Assistance, 94046 Créteil Cedex (FR)
(72) Inventeur: MAROUDIS, Pantelis, 94046 Créteil (FR); PINOTEAU, Jérémie, 94046 Créteil Cedex (FR)
(74) Mandataire: Delplanque, Arnaud

(57) **Abrégé**

Un dispositif de détection d'émotion d'un individu comprend :
- une unité de réception d'un signal vidéo (2) ;
- un module d'analyse du signal vidéo (6) conçu pour obtenir une première évaluation de l'état émotionnel de l'individu (Sv).

Le dispositif comporte également une unité de réception d'un signal audio (4), un module d'analyse du signal audio (12) conçu pour obtenir une deuxième évaluation de l'état émotionnel de l'individu (Sₜ ; Sₚ) et un module de combinaison (18) conçu pour combiner la première évaluation et la deuxième évaluation afin de déterminer une évaluation finale (S_{f}) de l'état émotionnel de l'individu.

Un procédé de détection d'émotion est également décrit.

## Description

### DOMAINE TECHNIQUE AUQUEL SE RAPPORTE L'INVENTION

La présente invention concerne de manière générale la détection d'émotion d'un individu.

Elle concerne plus particulièrement un dispositif et un procédé de détection d'émotion d'un individu.

### ARRIERE-PLAN TECHNOLOGIQUE

L'état émotionnel d'un individu peut conduire à des changements de comportement critiques, voire même dangereux si l'individu se trouve au volant d'un véhicule. Les véhicules automobiles comprennent aujourd'hui de multiples systèmes s'adaptant au conducteur et permettant d'améliorer la sécurité pendant la conduite. En particulier, des capteurs placés sur le volant permettent, à partir d'une mesure de la pression exercée sur le volant, d'estimer l'état de relâchement ou de crispation du conducteur. Cependant, les différentes habitudes et expériences de conducteurs peuvent conduire à une mauvaise appréciation de l'état émotionnel de l'individu.

On connaît notamment du document FR3040816 un dispositif permettant la détection de l'état physiologique et émotionnel du conducteur d'un véhicule automobile. Ce dispositif comporte une ou plusieurs caméras adaptées pour acquérir des images du conducteur selon différents angles de vue. Un module permet ensuite de générer un modèle tridimensionnel du visage du conducteur, un autre de générer une vue frontale du visage et un troisième module permet de détecter l'état émotionnel du conducteur du conducteur à partir des mouvements du visage identifiés sur une succession d'images. Grâce à une base de données répertoriant la correspondance entre l'état d'un individu et les mouvements de visage détectés, l'état émotionnel du conducteur peut ensuite être intégré à d'éventuelles consignes de conduite.

Cependant, ce dispositif ne permet la détection uniquement d'états émotionnels extrêmes, c'est-à-dire pour lesquels des mouvements du visage prononcés peuvent être identifiés et comparés aux mouvements préenregistrés dans la base de données.

### OBJET DE L'INVENTION

La présente invention propose d'améliorer la détermination de l'état émotionnel du conducteur d'un véhicule automobile.

Plus particulièrement, on propose selon l'invention un dispositif de détection d'émotion qui comprend une unité de réception d'un signal vidéo, un module d'analyse du signal vidéo conçu pour obtenir une première évaluation de l'état émotionnel de l'individu, caractérisé en ce qu'il comporte une unité de réception d'un signal audio, un module d'analyse du signal audio conçu pour obtenir une deuxième évaluation de l'état émotionnel de l'individu et un module de combinaison conçu pour combiner la première évaluation et la deuxième évaluation afin de déterminer une évaluation finale de l'état émotionnel de l'individu.

Ainsi, grâce à la présente invention, il est possible de détecter l'état émotionnel de l'individu, même s'il est modéré, grâce à l'analyse combinée des images du visage dudit individu et du signal audio provenant de cet individu.

D'autres caractéristiques non limitatives et avantageuses du dispositif de détection d'émotion conforme à l'invention, prises individuellement ou selon toutes les combinaisons techniquement possibles, sont les suivantes :
- le module d'analyse du signal audio comporte un module d'analyse du signal de voix de l'individu conçu pour obtenir la deuxième évaluation de l'état émotionnel de l'individu ;
- le module d'analyse du signal audio comporte un module d'analyse des mots contenus dans ledit signal, conçu pour obtenir la deuxième évaluation de l'état émotionnel de l'individu ;
- le module d'analyse du signal audio comporte un module d'analyse du signal de voix de l'individu conçu pour obtenir une troisième évaluation de l'état émotionnel de l'individu ;
- le module de combinaison est conçu pour combiner la première évaluation, la deuxième évaluation et la troisième évaluation de l'état émotionnel de l'individu ;
- le module de combinaison est conçu pour générer l'évaluation finale de l'état émotionnel d'un individu dès qu'une évaluation parmi lesdites évaluations est disponible ;
- le module d'analyse du signal vidéo contient un module de détection du mouvement des lèvres, conçu pour déceler si l'individu parle ;
- le module d'analyse du signal audio fonctionne lorsqu'un mouvement a été détecté par le module de détection du mouvement des lèvres ;
- le module de combinaison utilise un algorithme conçu pour obtenir l'évaluation finale de l'état émotionnel d'un individu ;
- le module de combinaison est conçu pour combiner la première évaluation et la deuxième évaluation en pondérant la première évaluation par un premier coefficient et la deuxième évaluation par un deuxième coefficient, le premier coefficient et le deuxième coefficient étant distincts ;
- le signal vidéo est acquis par une caméra infrarouge dirigée vers le visage de l'individu ; et
- ledit individu est le conducteur ou l'un des passagers d'un véhicule.

L'invention propose également un procédé de détection d'émotion, comprenant les étapes suivantes :
- obtention d'une première évaluation de l'état émotionnel de l'individu par analyse d'un signal vidéo,
- obtention d'une deuxième évaluation de l'état émotionnel de l'individu par analyse d'un signal audio, et
- détermination d'une évaluation finale de l'état émotionnel de l'individu par combinaison de la première évaluation et de la deuxième évaluation de l'état émotionnel de l'individu.

### DESCRIPTION DETAILLEE D'UN EXEMPLE DE REALISATION

La description qui va suivre en regard des dessins annexés, donnés à titre d'exemples non limitatifs, fera bien comprendre en quoi consiste l'invention et comment elle peut être réalisée.

Sur les dessins annexés :
- la figure 1 représente un exemple de système embarqué comprenant un dispositif de détection d'émotion conforme aux enseignements de l'invention ; et
- la figure 2 représente schématiquement l'habitacle d'un véhicule équipé d'un système embarqué tel que celui de la figure 1.

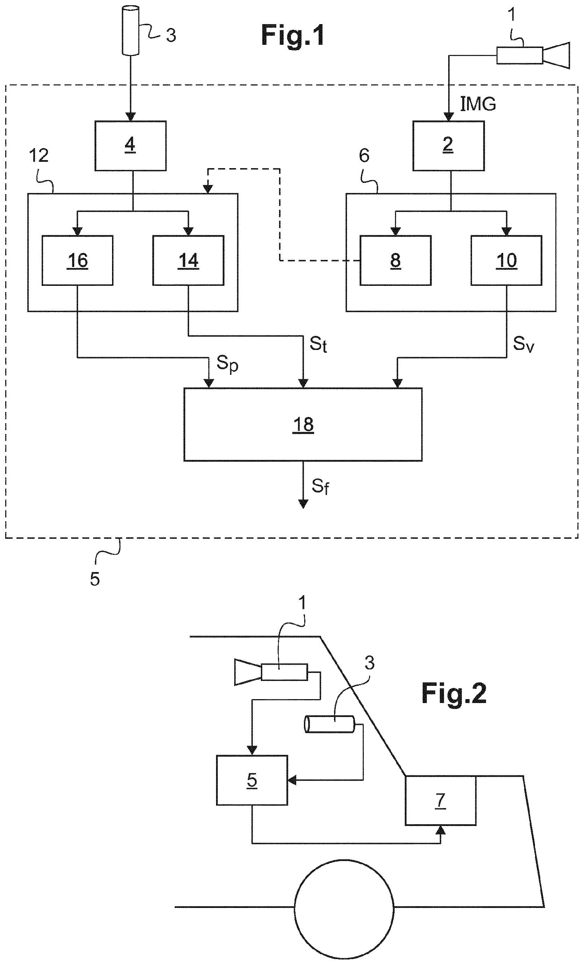

La figure 1 représente un exemple de système embarqué comprenant un dispositif de détection d'émotion 5, ici dans un véhicule automobile (tel que celui représenté sur la figure 2).

Le système embarqué de la figure 1 comprend un dispositif d'acquisition d'un signal vidéo 1, un dispositif d'acquisition d'un signal audio 3 et le dispositif de détection d'émotion 5 susmentionné.

Le dispositif d'acquisition du signal vidéo 1 comporte au moins une caméra, ici une caméra infrarouge. Le dispositif d'acquisition du signal vidéo peut comprendre en outre dans ce cas un élément émetteur de rayonnement infrarouge (non représenté ici) irradiant les zones de l'habitacle visées par la caméra susmentionnée (afin notamment que le dispositif d'acquisition puisse fonctionner même en cas de conduite nocturne). Chaque caméra est disposée dans l'habitacle du véhicule de manière à ne pas encombrer le champ de vision d'un conducteur assis. Chaque caméra est disposée de manière à capturer des images du visage du conducteur selon différents angles de vue.

Le dispositif d'acquisition du signal vidéo 1 est par exemple localisé dans l'habitacle du véhicule, dans une région voisine d'une partie supérieure du parebrise, de manière à acquérir des images de face du conducteur.

Le dispositif d'acquisition du signal audio 3 comporte au moins un microphone. Chaque microphone est disposé dans l'habitacle au plus proche de l'individu de manière à capturer clairement le son provenant dudit individu. Le dispositif d'acquisition du signal audio 3 est par exemple localisé dans une région voisine de l'appui-tête du siège de l'individu, de manière à acquérir le signal vocal dudit individu au plus proche de sa tête.

Le dispositif de détection d'émotion 5 contient un module de réception 2 du signal vidéo produit par le dispositif d'acquisition 1. Sur la base du signal vidéo reçu du dispositif d'acquisition 1, le module de réception 2 délivre un flux de données représentatives des images acquises par le dispositif d'acquisition du signal vidéo 1.

De même, le dispositif de détection d'émotion 5 contient également un module de réception 4 du signal audio produit par le dispositif d'acquisition 3. Le module de réception 4 délivre ainsi un flux de données représentatives du signal audio acquis par le dispositif d'acquisition 3.

Le dispositif de détection d'émotion 5 comprend ensuite un module d'analyse du signal vidéo 6. Ce module est conçu pour recevoir les données provenant du module de réception du signal vidéo 2 puis les analyser afin d'obtenir une première évaluation de l'état émotionnel de l'individu Sv. Ce module comporte ici deux éléments : un module de détection du mouvement des lèvres 8 et un module de détermination de la première évaluation de l'état émotionnel de l'individu 10.

L'évaluation de l'état émotionnel de l'individu peut en pratique être formée d'une pluralité de valeurs, par exemple deux valeurs relatives à l'émotion détectée. Selon une possibilité de réalisation, une première valeur porte sur la qualification de l'état émotionnel, par exemple la joie, la colère, la surprise, *etc.* et une deuxième valeur est relative à l'intensité de cette émotion identifiée. Dans la suite de cette description, le terme « évaluation » regroupera la première valeur et la deuxième valeur, sans les distinguer explicitement.

Le module de détermination de la première évaluation de l'état émotionnel de l'individu 10 comprend un algorithme de reconnaissance faciale. Ledit module de détermination 10 comprend également un algorithme de suivi capable, à partir du signal vidéo représenté par les données transmises par l'unité de réception du signal vidéo 2, d'établir la première évaluation susmentionnée de l'état émotionnel de l'individu S_{V}. On pourra par exemple se référer à l'article « Real time facial expression recognition in video using support vector machines » de Michel et El Kaliouby, Proceedings of the 5th international conférence on Multimodal interfaces, ACM, 2003, pour plus de détails à ce sujet.

Le module de détection du mouvement des lèvres 8 est quant à lui conçu pour identifier la région des lèvres du conducteur au sein des images représentées par les données reçues au sein du module de réception 4 et pour détecter un mouvement éventuel de ses lèvres (indicatif du fait que le conducteur parle).

Le dispositif de détection d'émotion 5 comprend également un module d'analyse du signal audio 12. Le fonctionnement de ce module 12 est ici enclenché lorsqu'un mouvement de lèvres a été détecté par le module de détection 8 susmentionné. Le module d'analyse du signal audio 12 comporte ici deux éléments : un module d'analyse du signal de voix de l'individu 14 et un module d'analyse sémantique du signal de voix 16 conçu pour analyser les mots contenus dans le signal audio.

Le module d'analyse sémantique du signal de voix 16 est conçu pour obtenir une deuxième évaluation de l'état émotionnel d'un individu Sₚ. Un logiciel de reconnaissance des mots et expressions permet d'identifier le contenu de la conversation. Un algorithme de traitement du langage mis en place dans ce module 16 permet ensuite d'établir une concordance lexicale entre les mots identifiés dans la conversation et ceux répertoriés dans l'algorithme de traitement. L'algorithme de traitement associe ensuite des probabilités aux différents mots-clés mis en évidence pour caractériser l'état émotionnel de l'individu. L'algorithme procède alors à une catégorisation et une hiérarchisation des différentes émotions détectées. Pour cela, un algorithme de détection des émotions contenu dans le module 16 calcule le poids associé à chaque émotion en fonction des mots détectés (nombre de mots liés à l'émotion, intensité des mots identifiés) et permet de classer les émotions détectées par niveau. L'algorithme extrait ensuite l'estimation la plus pertinente qui correspond à la deuxième évaluation de l'état émotionnel de l'individu Sₚ. On pourra par exemple se référer aux articles « Speech emotion récognition » de Ingale et Chaudhari, International Journal of Soft Computing and Engineering (IJSCE) 2.1 (2012) : 235-238 et « Emotion détection from text » de Shivhare et Khethawat, arXiv preprint arXiv : 1205.4944 (2012), pour plus de détails quant à l'analyse sémantique du signal de voix.

Le module d'analyse du signal de voix de l'individu 14 est conçu pour obtenir une troisième évaluation de l'état émotionnel de l'individu Sₜ à partir de l'analyse du ton de la voix dudit individu. Le ton de la voix d'un individu retranscrit également l'état émotionnel d'un individu. L'extraction des caractéristiques statistiques de la modulation du signal de voix à partir d'un algorithme permet alors d'estimer la troisième évaluation susmentionnée de l'état émotionnel de l'individu Sₜ. On pourra par exemple se référer à l'article « Analysis of Emotionally Salient Aspects of Fundamental Frequency for Emotion Détection » de Busso, Lee et Narayanan, IEEE Transactions on Audio, Speech, and Language Processing, vol.17, no 4, pp. 582-596, Mai 2009 pour plus de détails à ce sujet.

Le dispositif de détection d'émotion 5 comporte également un module de combinaison 18 conçu pour combiner la première évaluation S_{V}, la deuxième évaluation Sₚ et la troisième évaluation Sₜ. Le module 18 repose sur l'utilisation d'un algorithme de fusion qui permet de générer en sortie, sur la base de la première évaluation S_{V}, de la deuxième évaluation Sₚ et de la troisième évaluation Sₜ, une évaluation finale de l'état émotionnel de l'individu S_{f}. Selon une possibilité de réalisation, l'algorithme de fusion peut être choisi de sorte que le module 18 fonctionne (i.e. délivre l'évaluation finale susmentionnée) dès que l'une des évaluations au moins parmi les trois (S_{V}, Sₚ, Sₜ) est disponible.

L'algorithme de fusion pourra par exemple reposer sur une machine à vecteurs de support (ou SVM), un réseau de neurones artificiels, un arbre de décision, un réseau Bayésien ou une méthode statistique, qui correspondent à un ensemble de techniques numériques connues et qui ne seront pas décrites plus en détail.

Dans le cas où une seule évaluation parmi les trois (S_{V}, Sₚ, Sₜ) est disponible, l'algorithme de fusion sera adapté pour utiliser directement la seule évaluation disponible en tant qu'évaluation finale S_{f}.

Selon un autre mode de réalisation envisageable, le module de combinaison 18 peut générer l'évaluation finale de l'état émotionnel de l'individu S_{f} au moyen d'une pondération des trois évaluations S_{V}, Sₚ et Sₜ. Ledit module 18 est alors conçu pour combiner la première évaluation S_{V} en la pondérant avec un premier coefficient α, la deuxième évaluation Sₚ en la pondérant avec un deuxième coefficient β et la troisième évaluation Sₜ avec un troisième coefficient γ. On peut envisager par exemple dans ce cas de mettre automatiquement à 0 le coefficient correspondant à l'une des évaluations si cette évaluation n'est pas disponible.

Les coefficients de pondération α, β, γ sont déterminés en tenant compte de la fiabilité relative de la première évaluation S_{V}, de la deuxième évaluation Sₚ et de la troisième évaluation Sₜ. L'estimation de la fiabilité des évaluations prend en compte la performance générale des différentes méthodes d'évaluation ainsi qu'une estimation de la qualité de chaque évaluation obtenue au vu des données reçues en entrée.

On a représenté sur la figure 2 un exemple d'intégration du système embarqué comprenant le dispositif de détection d'émotion 5 dans un véhicule. On peut envisager que l'évaluation finale de l'état émotionnel de l'individu S_{f} soit transmise à un module de commande 7.

On peut prévoir par exemple que le module de commande 7 mémorise des valeurs seuils de niveau d'état émotionnel au delà desquelles le module de commande 7 déclenche la communication d'une alerte à l'individu et/ou émet les commandes nécessaires pour une conduite en toute sécurité. Dans le cas mentionné ci-dessous où l'évaluation est représentée par deux valeurs, le module de commande reçoit la première valeur et la deuxième valeur concernant l'émotion détectée. Les alertes transmises au conducteur ne sont pertinentes que pour certains états émotionnels prédéfinis, comme la colère par exemple, et auxquels sont associées des intensités élevées.

Par exemple, on peut envisager que si l'évaluation finale de l'état émotionnel de l'individu S_{f} a une intensité comprise entre deux valeurs prédéterminées ε₁ et ε₂ pour l'un des états émotionnels prédéfinis, le module de commande 7 génère un message automatique mettant en garde l'individu. On peut aussi envisager que si l'évaluation finale de l'état émotionnel de l'individu S_{f} a une intensité supérieure à une valeur seuil ε_{f}, cas correspondant à un état émotionnel dangereux vis-à-vis de la conduite d'un véhicule, le module de commande 7 active un mode de conduite autonome du véhicule.

## Revendications

1. Dispositif de détection d'émotion d'un individu comprenant :
- une unité de réception d'un signal vidéo (2) ;
- un module d'analyse du signal vidéo (6) conçu pour obtenir une première évaluation de l'état émotionnel de l'individu (Sv) ;
**caractérisé en ce qu'**il comporte une unité de réception d'un signal audio (4), un module d'analyse du signal audio (12) conçu pour obtenir une deuxième évaluation de l'état émotionnel de l'individu (Sₜ ; Sₚ) et un module de combinaison (18) conçu pour combiner la première évaluation (S_{V}) et la deuxième évaluation (Sₜ ; Sₚ) afin de déterminer une évaluation finale (S_{f}) de l'état émotionnel de l'individu.

2. Dispositif de détection d'émotion d'un individu selon la revendication 1, dans lequel le module d'analyse du signal audio (12) comporte un module d'analyse du signal de voix de l'individu (14) conçu pour obtenir la deuxième évaluation de l'état émotionnel de l'individu (Sₜ).

3. Dispositif de détection d'émotion d'un individu selon la revendication 1, dans lequel le module d'analyse du signal audio (12) comporte un module d'analyse des mots contenus dans ledit signal (16), conçu pour obtenir la deuxième évaluation de l'état émotionnel de l'individu (Sₚ).

4. Dispositif de détection d'émotion d'un individu selon la revendication 3, dans lequel le module d'analyse du signal audio (12) comporte un module d'analyse du signal de voix de l'individu (14) conçu pour obtenir une troisième évaluation de l'état émotionnel de l'individu (Sₜ).

5. Dispositif de détection d'émotion d'un individu selon la revendication 4, dans lequel le module de combinaison (18) est conçu pour combiner la première évaluation (S_{V}), la deuxième évaluation (Sₚ) et la troisième évaluation (Sₜ) de l'état émotionnel de l'individu.

6. Dispositif de détection d'émotion d'un individu selon l'une des revendications 1 à 5, dans lequel le module de combinaison (18) est conçu pour générer l'évaluation finale (S_{f}) de l'état émotionnel d'un individu dès qu'une évaluation parmi lesdites évaluations (S_{V} ; Sₜ ; Sₚ) est disponible.

7. Dispositif de détection d'émotion d'un individu selon l'une des revendications 1 à 6, dans lequel le module d'analyse du signal vidéo (6) contient un module de détection du mouvement des lèvres (8).

8. Dispositif de détection d'émotion d'un individu selon la revendication 7, dans lequel le module d'analyse du signal audio (12) fonctionne lorsqu'un mouvement a été détecté par le module de détection du mouvement des lèvres (8).

9. Dispositif de détection d'émotion d'un individu selon l'une des revendications 1 à 8, dans lequel le module de combinaison (18) utilise un algorithme conçu pour obtenir l'évaluation finale (S_{f}) de l'état émotionnel d'un individu.

10. Dispositif de détection d'émotion d'un individu selon l'une des revendications 1 à 9, dans lequel le module de combinaison (18) est conçu pour combiner la première évaluation (S_{V}) et la deuxième évaluation (Sₜ ; Sₚ) en pondérant la première évaluation par un premier coefficient α et la deuxième évaluation par un deuxième coefficient (β ; γ), le premier coefficient α et le deuxième coefficient (β ; γ) étant distincts.

11. Dispositif de détection d'émotion d'un individu selon l'une des revendications 1 à 10, dans lequel le signal vidéo est acquis par une caméra infrarouge (1) dirigée vers le visage de l'individu.

12. Dispositif de détection d'émotion d'un individu selon l'une des revendications 1 à 11, dans lequel ledit individu est le conducteur ou l'un des passagers d'un véhicule.

13. Procédé de détection d'émotion comportant des étapes de :
- obtention d'une première évaluation (Sv) de l'état émotionnel de l'individu par analyse d'un signal vidéo,
**caractérisé en ce qu'**il comporte également les étapes de :
- obtention d'une deuxième évaluation (Sₜ ; Sₚ) de l'état émotionnel de l'individu par analyse d'un signal audio, et
- détermination d'une évaluation finale (S_{f}) de l'état émotionnel de l'individu par combinaison de la première évaluation (S_{V}) et de la deuxième évaluation (Sₜ ; Sₚ) de l'état émotionnel de l'individu.
